# EUROPEAN PATENT APPLICATION

(11) **EP 4 759 844 A1**
(43) Date of publication of application: **17.06.2026**
(21) Application number: 24853703.7
(22) Date of filing: 09.08.2024
(51) Int. Cl.: C08F 293/00, C08F 20/06

(54) **LUBRICATING COMPOSITION COMPRISING BLOCK COPOLYMER AND USE THEREOF**

(30) Priority: 11.08.2023 CN 202311010940
(71) Applicant: Pleryon Therapeutics (Hangzhou) Limited, Hangzhou, Zhejiang 310064 (CN)
(72) Inventor: SUN, Zhexun, Shenzhen, Guangdong 518000 (CN); YU, Yu, Shenzhen, Guangdong 518000 (CN)
(74) Representative: Murgitroyd & Company
(86) International application number: PCT/CN2024/111105
(87) International publication number: WO 2025/036294

(57) **Abstract**

A lubricating block copolymer and a use thereof, and a composition comprising the lubricating block copolymer and a use thereof. Also provided is a method for imparting a suitable level of lubrication to biological tissue. The method comprises: bringing biological tissue into contact with said block copolymer and/or said composition so as to impart a suitable level of lubrication.

## Description

### TECHNICAL FIELD

The present application relates to the field of biomedicine, and in particular to a lubricating composition of a block copolymer and use thereof.

### BACKGROUND

Lubricin is a glycosylated protein present in synovial fluid, which plays a key role in joint boundary lubrication and prevention of osteoarthritis. Lubricin reduces the coefficient of friction (COF) of articular cartilage in the boundary mode by up to 70% (Gleghorn, J.P. et al., J. Orthop. Res. 2009, 27(6), 77). This efficient lubricating ability stems from the structure of lubricin: the central mucin-like domain of lubricin is composed of an extensively glycosylated core protein, which attracts and retains water in the vicinity of the molecule; the C-terminus of lubricin binds this protein to the cartilage surface (Zappone, B. et al., Langmuir 2008, 24(4), 1495). This structure is critical for boundary-mode lubrication of articular cartilage, as denaturation of any domain of lubricin results in partial or complete loss of lubricating ability.

In developed countries and regions, over 50 million people suffer from osteoarthritis (OA), and this number is expected to rise as median age and life expectancy increase. In the United States alone, the annual economic impact of treating osteoarthritis exceeds $30 billion. The economic burden, along with other factors (i.e., quality of life, lost work hours, etc.), motivates the development of more effective treatments.

Current treatments for osteoarthritis (OA) include non-steroidal anti-inflammatory drugs, intra-articular corticosteroid injections, and chondroitin sulfate or glucosamine supplements. However, they have little or no effect on the progression of the disease. A recent approach to treating OA is intra-articular injection of the natural synovial fluid glycosaminoglycan, hyaluronic acid (HA) (e.g., Mabuchi et al., (1994) J. Biomed. Mat. Res. 28:865-70). HA is known to improve the viscosity of synovial fluid (e.g., viscosupplementation) to reduce the coefficient of friction in hydrodynamic-mode lubrication (e.g., Tadmor et al., (2002) J. Biomed. Mat. Res. 61:514-23). Another major lubricating component in synovial fluid is lubricin, which is a high-molecular-weight glycoprotein that can reduce the coefficient of friction in boundary-mode lubrication.

In damaged cartilage, it is well known that lubricin production by chondrocytes is diminished, and boundary-mode lubrication is reduced. Natural lubricants in nature (such as proteoglycan aggregates and mucins (e.g., lubricin)) maintain natural surfaces as hydrophilic. In rat disease models, intra-articular injection of supplemental lubricin and a truncated recombinant lubricin construct LUB:1 slowed the progression of OA (e.g., Jay et al., (2010) Arthritis Rheum. 62:2382-91; Flannery et al., (2009) Arthritis Rheum. 60:840-7). However, to date, large-scale recombinant production of both lubricin and LUB:1 remains challenging, due to the multiple amino acid repeats in the core structure of the aforementioned proteins and their high degree of glycosylation (e.g., Jay (2004) Curr. Opin. Orthop. 15:355-359; Jones et al., (2007) J. Orthop. Res. 25:283-292). In cases where direct bone-to-bone contact may occur in the advanced stages of osteoarthritis, an effective bone lubricant is additionally required. Therefore, an effective lubricant capable of providing a boundary lubrication effect equivalent or similar to that of lubricin or LUB:1 would represent a significant advancement in this field.

### SUMMARY

The present application provides the design, synthesis, and use of a special block copolymer having a lubricin-mimetic structure, which provides a significant lubricating ability under boundary-mode lubrication conditions. In certain embodiments, the block copolymer contains a lubricating block (e.g., Mn ~ 200 kDa) that mimics the mucin-like domain of lubricin and a smaller cartilage-binding block (e.g., Mn ~ 3 kDa) that mimics the hemopexin-like domain. As disclosed below in the present application, applying this type of polymer to a biological tissue results in a significant reduction in the coefficient of friction (COF) compared to an untreated control.

In one aspect, the present application provides a block copolymer having the following structure: 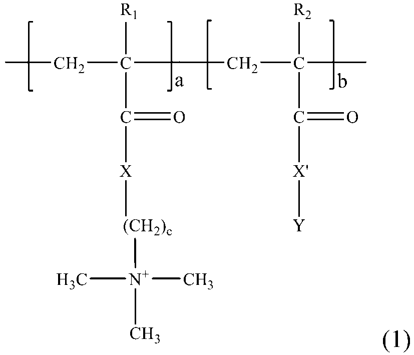 wherein: R1 and R2 are each independently selected from hydrogen and methyl; X and X' are each independently selected from -NR'-, -O-, and a bond, wherein R' is selected from a hydrogen atom and a group having at least 1 and at most 6 carbon atoms; Y is selected from a polyalkylene glycol, a saccharide, and a polyol; subscript a is an integer of at least 3, subscript b is at least twice a, and subscript c is an integer of at least 1; according to the laws of chemistry, the block copolymer is terminated at each terminus with a terminal group, and the total positive charge of quaternary ammonium groups in the copolymer is counterbalanced by an equal magnitude of total negative charge provided by anions associated with the quaternary ammonium groups.

In certain embodiments, the anion is a chloride ion, a bromide ion, or an iodide ion. In some embodiments, the anion is a chloride ion.

In certain embodiments, subscript b is at least twice a. In certain embodiments, subscript b is an integer greater than 40. In certain embodiments, subscript b is an integer of at least 50. In certain embodiments, subscript b is an integer of at least 100. In certain embodiments, subscript b is an integer of 200-400, and subscript a is an integer of 20-96. In certain embodiments, subscript b is 400, and subscript a is 24.

In certain embodiments, Y comprises a polyalkylene glycol. In certain embodiments, the polyalkylene glycol comprises polyethylene glycol. In certain embodiments, the saccharide comprises a monosaccharide. In certain embodiments, the saccharide comprises at least two monosaccharide units. In certain embodiments, the polyalkylene glycol, the saccharide, or the polyol is composed of at least 2 and at most 500 monomer units. In certain embodiments, the polyalkylene glycol, the saccharide, or the polyol is composed of at least 2 and at most 200 monomer units. In certain embodiments, the polyalkylene glycol, the saccharide, or the polyol is composed of at least 2 and at most 100 monomer units. In certain embodiments, the polyalkylene glycol, the saccharide, or the polyol is composed of at least 2 and at most 20 monomer units. In certain embodiments, at least one of the terminal groups is sulfhydryl.

In another aspect, the present application provides a composition, comprising the block copolymer described in the present application.

In another aspect, the present application provides use of the block copolymer described in the present application and/or the composition described in the present application in enhancing the lubricity of a biological tissue or imparting an appropriate level of lubricity to a biological tissue.

In another aspect, the present application provides a method for imparting an appropriate level of lubricity to a biological tissue, comprising bringing the biological tissue into contact with the block copolymer described in the present application and/or the composition described in the present application to impart the appropriate lubricity.

In another aspect, the present application provides use of the block copolymer and/or the composition in treating dry eye disease.

In another aspect, the present application provides use of the block copolymer and/or the composition in preparing a medicament for treating dry eye disease.

In certain embodiments, the biological tissue is selected from a joint, a bone, an ocular tissue, a nasal tissue, a tendon, a tendon sheath, and a vaginal tissue.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the description in the drawings and specification of the present application is provided only for the purpose of illustration rather than limitation.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. A brief description of the drawings is as follows:
FIG. 1 shows a general representation of an exemplary diblock copolymer of the present application, with a binding block moiety and a lubricating block moiety identified.
FIG. 2 shows a schematic diagram of the degradation of the block copolymer of the present application, the degradation product of which may include a naturally occurring compound.
FIGs. 3A-C show the PDI test results of the block copolymers of the present application, the PDI values of which exhibit a certain correlation with the type of counterion.
FIG. 4 shows the lubricating ability of an exemplary block copolymer of the present application, which reduces the coefficient of friction by at least 60% under different boundary conditions.
FIG. 5 shows the biocompatibility of key intermediates of the block copolymer of the present application, where the cytotoxicity of the intermediates exhibits a certain correlation with the type of counterion.
FIG. 6 shows the stability of the block copolymers of the present application, indicating that the inclusion of a methyl structure on the main chain can effectively inhibit the generation of degradation byproducts during moist heat sterilization.
FIG. 7 shows the therapeutic effect of an exemplary block copolymer of the present application, which effectively reduced the corneal staining score and restored it to a normal level in a mouse model of dry eye disease.
FIG. 8 shows the therapeutic effect of the exemplary block copolymer of the present application, which effectively reduced the expression levels of some inflammatory factors in the mouse model of dry eye disease.

### DETAILED DESCRIPTION

The implementation of the invention of the present application is described below with reference to specific embodiments, and those skilled in the art can easily understand other advantages and effects of the invention of the present application from the disclosure of this specification.

### Definitions of Terms

In the present application, the term "block copolymer", also known as a mosaic copolymer, generally refers to a special polymer prepared by connecting two or more polymer segments with different properties. For example, the block copolymer described in the present application may comprise a mucin-like domain and a C-terminal hemopexin-like (PEX-like) domain. For example, the block copolymer described in the present application comprises a binding domain and a lubricating domain. In the present application, the term "copolymer" means that at least two polymer blocks are present. The copolymer may be, for example, a diblock copolymer, a triblock copolymer, a tetrablock copolymer, or a multiblock copolymer with even more blocks. The copolymer may, for example, comprise a polymer block containing positively or negatively charged side groups, and a polymer block containing non-ionic hydrophilic side groups, particularly hydrophilic groups containing ether and/or hydroxyl functional groups. In cases where the side groups are polymers, the copolymer can be further classified as a graft brush copolymer.

In the present application, the term "polymer dispersity index" or "PDI", also known as polydispersity index or heterogeneity index, is generally used to describe the molecular weight distribution of a polymer and is the ratio of the weight-average molecular weight to the number-average molecular weight. The value of PDI is greater than or equal to 1. The larger the PDI, the broader the molecular weight distribution; the smaller the PDI, the more uniform the molecular weight distribution.

In the present application, the term "pharmaceutically acceptable carrier" or equivalent terminology refers to a pharmaceutically acceptable material, composition, or carrier, which may be a liquid (diluent or excipient) or a solid filler. In the present application, "pharmaceutically acceptable" refers to compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio. In a pharmaceutical composition, the compound is typically dispersed in a physiologically acceptable carrier, either by mixing (e.g., mixing in a solid form with a solid carrier) or by dissolution or emulsification in a liquid carrier. The carrier should be compatible with the other components of the formulation and physiologically safe for the subject. Any carrier known in the art is suitable for the present application, depending on the mode of administration. Some examples of suitable carriers include aqueous solutions, gelatin, fatty acids (e.g., stearic acid) and salts thereof, talc, vegetable fats or oils, gums and glycols, starch, dextran, etc. The terms "sufficient amount", "therapeutically effective amount", and "effective amount", used interchangeably in the present application, refer to an amount of the copolymer composition of the present application that is sufficient to achieve the following results: providing adequate lubricity to a biological tissue, or preventing the development, recurrence, or onset of a disease or condition (e.g., osteoarthritis) or one or more symptoms thereof, enhancing or improving the prophylactic effect of another therapy, reducing the severity and duration of a disease or disorder, alleviating one or more symptoms of the disease or disorder, preventing the development of the disease or disorder, and/or enhancing or improving the efficacy of an additional treatment.

### Detailed Description of the Present Disclosure

The block copolymer and/or the composition comprising the block copolymer described in the present application has one or more of the following functions or advantages:
(1) when the counterion is Cl, it has ameliorated cytotoxicity and improved biocompatibility;
(2) when the counterion is Cl, it has a uniform molecular weight distribution and a relatively small PDI (polymer dispersity index);
(3) when the counterion is Cl, its synthetic route is shorter, and its synthesis is convenient;
(4) it possesses a lubricating ability for a biological tissue; and
(5) when the main chain contains methyl, it exhibits greater stability.

The block copolymer described in the present application has ameliorated cytotoxicity and improved biocompatibility compared to other combinations. The block copolymer described in the present application has a structure similar to that of the naturally occurring compound choline, and no other analogous structures have been found in natural biological systems. As for the block copolymer described in the present application, as the hydrocarbon chain increases, the hydrophobicity of the amino group increases, leading to amphiphilicity commonly found in cell lysis reagents.

In general, as the hydrocarbon chain increases, the increase in hydrophobicity/amphiphilicity of the compound increases the challenge of synthesis and leads to a broader molecular weight distribution. In contrast, the block copolymer described in the present application has a PDI close to 1, exhibiting a uniform molecular weight distribution.

In one aspect, the present application provides a series of block copolymers having the structure as shown below:

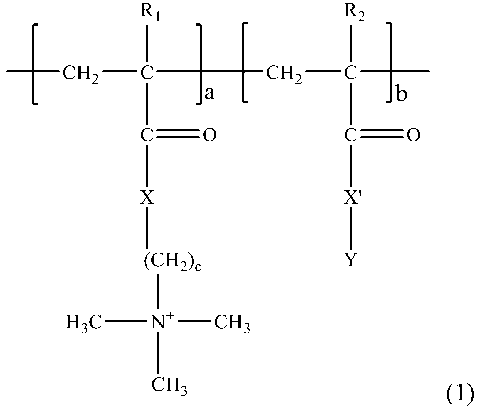

In formula (1), the variables X and X' are each independently selected from -NR'-, -O-, and a bond, wherein R' is selected from a hydrogen atom and a hydrocarbon group having at least 1 and at most 6 carbon atoms (selected from an R group). In certain embodiments, R' is particularly selected from a hydrogen atom, methyl, ethyl, n-propyl, isopropyl, n-butyl, sec-butyl, isobutyl, and tert-butyl, or is a more specific selection.

In another aspect, the present application provides a series of block copolymers having a structure represented by formula (1), wherein: R1 and R2 are selected from hydrogen and methyl, and R1 and R2 are not both methyl; X and X' are each independently selected from -NR'-, -O-, and a bond, wherein R' is selected from a hydrogen atom and a group having at least 1 and at most 6 carbon atoms; Y is selected from a polyalkylene glycol, a saccharide, and a polyol; subscripts a and b are each independently an integer of at least 3, and subscript c is an integer of at least 1; according to the laws of chemistry, the block copolymer is terminated at each terminus with a terminal group, and the total positive charge of quaternary ammonium groups in the copolymer is counterbalanced by an equal magnitude of total negative charge provided by anions associated with the quaternary ammonium groups. In some embodiments, the anion is chloride, bromide, or iodide. In some embodiments, the anion is chloride.

In certain embodiments, R1 and R2 are both hydrogen. In certain embodiments, R1 is hydrogen, and R2 is methyl. In certain embodiments, R1 is methyl, and R2 is hydrogen. In certain embodiments, R1 is methyl, and R2 is methyl.

In another aspect, the present application provides a series of block copolymers having a structure represented by formula (21): 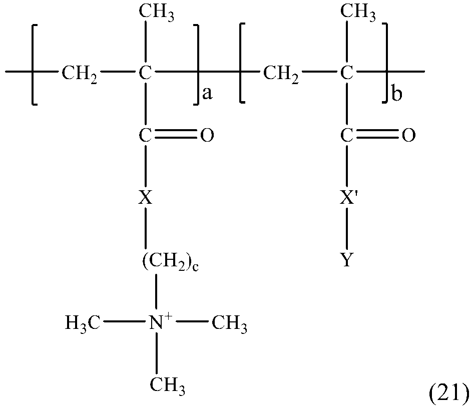 wherein: X and X' are each independently selected from -NR'-, -O-, and a bond, wherein R' is selected from a hydrogen atom and a group having at least 1 and at most 6 carbon atoms; Y is selected from a polyalkylene glycol, a saccharide, and a polyol; subscripts a and b are each independently an integer of at least 3, and subscript c is an integer of at least 1; according to the laws of chemistry, the block copolymer is terminated at each terminus with a terminal group, at least one of the terminal groups is sulfhydryl, and the total positive charge of quaternary ammonium groups in the copolymer is counterbalanced by an equal magnitude of total negative charge provided by anions associated with the quaternary ammonium groups. In some embodiments, the anion is chloride, bromide, or iodide. In some embodiments, the anion is chloride.

In certain embodiments, in formula (1), the variable Y is or comprises a polyalkylene glycol group. The polyalkylene glycol group may be conveniently represented by the following structure: (-CR'₂CR'₂O-)ₙR', wherein for each instance of R', R' is independently selected from a hydrogen atom and a hydrocarbon group (e.g., methyl or ethyl), and n is at least 2, 3, 4, 5, or 6 and at most, for example, 8, 9, 10, 12, 15, 20, 25, 30, 35, 40, 50, 60, 70, 80, 90, 100, 200, 300, 400, or 500, or n is within a range defined by any two of the aforementioned values, wherein each of the aforementioned values corresponds to the number of alkylene oxide (-CR'₂CR'₂O-) monomer units. In a specific embodiment, the polyalkylene glycol is a polyethylene glycol or polypropylene glycol group. Furthermore, the polyalkylene glycol group may or may not be incorporated into the copolymer, for example, a copolymer containing polyethylene glycol and an anionic polymer moiety, such as poly(acrylic acid) (PAA), polyglutamic acid, or polyaspartic acid.

In certain embodiments, in formula (1), the variable Y is or comprises a saccharide group. In the present application, the term "saccharide" includes monosaccharides (containing one monosaccharide unit) and saccharides containing at least or more than two monosaccharide units, such as disaccharides, trisaccharides, oligosaccharides (e.g., at least 4 and at most 20, 30, 40, 50, or 60 monosaccharide units), and polysaccharides (typically more than 60, 70, or 80 monosaccharide units, and up to, for example, 100, 200, 300, 400, 500, or 1000 monosaccharide units). Saccharides may also be derivatized in such a manner (e.g., esterification, etherification, amination, or halogenation) that the derivatized form can still be reasonably classified as a saccharide by those skilled in the art. Some examples of monosaccharides include glucose, galactose, fructose, mannose, sialic acid, glucosamine, N-acetylglucosamine, and galacturonic acid. Some examples of disaccharides include lactose, sucrose, maltose, trehalose, cellobiose, and mannobiose. Some examples of oligosaccharides include fructooligosaccharides (FOS), galactooligosaccharides (GOS), and mannooligosaccharides (MOS). Some examples of polysaccharides include dextran, dextran sulfate, starch (e.g., amylose or amylopectin), cellulose, hemicellulose, polysialic acid, pectin, glycogen, mannan, galactomannan, xylan, pullulan, xanthan gum, carrageenan, guar gum, polygalacturonic acid, poly(N-acetylgalactosamine), heparin, hyaluronic acid, and chondroitin sulfate. In certain embodiments, the saccharide is selected to have an overall anionic charge, such as those saccharides having carboxylic acid, carboxylate, sulfate, or sulfonate groups. The saccharide group may or may not be incorporated into the copolymer, for example, a copolymer containing an oligosaccharide moiety and an oligopeptide or poly(acrylic acid) moiety. In certain embodiments, the copolymer containing a saccharide contains a saccharide moiety and an anionic polymer moiety, such as poly(acrylic acid) (PAA), polyglutamic acid, or polyaspartic acid.

In certain embodiments, in formula (1), the variable Y comprises a polyol group. In the present application, the term "polyol" generally refers to a non-saccharide group having multiple (i.e., at least 2, 3, or 4) hydroxyl groups. The polyol may be, for example, a sugar alcohol or a polyhydric alcohol. Some examples of sugar alcohols include erythritol, xylitol, mannitol, glycerol, and sorbitol. In certain embodiments, the polyol is a polymer containing hydroxyl. The saccharide or polyol (Y) is typically attached to the block copolymer of formula (1) via one hydroxyl group in its deprotonated form, wherein X' may represent the oxygen atom of the Y group; or X' may represent a bond, and Y represents a saccharide or polyol bound via its oxygen atom to the indicated C(O) group; or X' is - NR'-, and Y represents a saccharide or polyol bound via its carbon atom to the -NR'- group. In certain embodiments, the polymer containing hydroxyl may comprise, for example, at least 2, 3, 4, 5, or 6 and at most, for example, 8, 9, 10, 12, 15, 20, 25, 30, 35, 40, 50, 60, 70, 80, 90, 100, 200, 300, 400, or 500 monomer units. Some examples of such polymers include polyvinyl alcohol, poly(hydroxyethyl methacrylate), and poly(hydroxypropyl methacrylate). The polyol group may or may not be incorporated into the copolymer, for example, a polyvinyl alcohol-poly(acrylic acid) (PVA-PAA) copolymer. In certain embodiments, the copolymer containing a polyol contains a polyol moiety and an anionic moiety, which may be an anionic polymer moiety, such as poly(acrylic acid) (PAA), polyglutamic acid, or polyaspartic acid.

In certain embodiments, in formula (1), subscripts a and b are each independently an integer of at least 3, 4, 5, 6, 7, 8, 9, 10, 12, 15, 20, 25, 30, 35, 40, or 50. In certain embodiments, subscripts a and b are each independently no more than 30, 40, 50, 60, 70, 80, 90, 100, 150, 200, 250, 300, 350, 400, 450, 500, 600, 700, 800, 900, 1000, 1200, 1500, 1800, or 2000. In certain embodiments, the selected subscript a is less than subscript b. For example, subscript a may be in a range of 3-10, 3-20, 3-30, or 3-40, while subscript b is in a range of 30-500, 40-500, 50-500, or 60-500. In other embodiments, the selected subscript a is greater than subscript b. For example, subscript a may be in a range of 30-500, 40-500, 50-500, or 60-500, while subscript b is in a range of 3-10, 3-20, 3-30, or 3-40.

In certain embodiments, in formula (1), subscript a may be an integer of at least 3, and subscript b may be at least twice a. In certain embodiments, subscript b may be at least 5 times a. In certain embodiments, subscript b may be at least 10 times a. In certain embodiments, subscript b may be at least 20 times a. In certain embodiments, subscript b may be at least 50 times a. In certain embodiments, subscript b may be at least 100 times a. In certain embodiments, subscript b may be at least 200 times a.

In certain embodiments, in formula (1), subscript a may be an integer of at least 3, and subscript b may be an integer greater than 40. In certain embodiments, subscript b may be an integer of at least 50. In certain embodiments, subscript b may be an integer of at least 100. In certain embodiments, subscript b may be an integer of at least 200. In certain embodiments, subscript b may be an integer of at least 300. In certain embodiments, subscript b may be an integer of at least 400.

In certain embodiments, subscript a may be in a range of 3-10, 3-20, 3-30, or 3-40, and subscript b may be in a range of 40-500, 50-500, or 60-500. In certain embodiments, subscript a may be in a range of 3-30, and subscript b may be in a range of 40-500. In certain embodiments, subscript a may be in a range of 20-30, and subscript b may be in a range of 300-500. In certain embodiments, subscript a may be an integer of 10-100, and subscript b may be an integer of 100-500. In certain embodiments, subscript a may be an integer of 20-96, and subscript b may be an integer of 200-400. In certain embodiments, subscript a may be 24, and subscript b may be 400.

In formula (1), subscript c is an integer of at least 1. In various embodiments, c is exactly 1, 2, 3, 4, 5, 6, 7, 8, 9, 10, 11, or 12, or c is within a range defined by any two of the aforementioned values. The value of c corresponds to the number of methylene groups to which c corresponds. That is, when subscript c is 1, a CH₂ linker is present between X and the quaternary ammonium group shown in formula (1); and when subscript c is 2, a CH₂CH₂ linker is present between X and the quaternary ammonium group shown in formula (1).

Although not shown in formula (1), the structure represented by formula (1) necessarily (i.e., by the laws of chemistry) comprises a terminal group at each of the two termini of the copolymer. The terminal groups are each independently selected from, for example, a hydrogen atom, a hydrocarbon group R or R', or a heteroatom-containing group (e.g., -OH or -OCH3), a nitrile-containing alkyl group (e.g., provided by a free-radical initiator or chain-transfer agent), a sulfhydryl group (-SH), or a dithioester group (provided by a RAFT chain-transfer agent). In some embodiments, at least one of the terminal groups is sulfhydryl.The terminal groups typically correspond to the groups initially present in the precursor reactants used to synthesize the block copolymer, and therefore the type of terminal group generally depends on the chemical substances used to synthesize the block copolymer. However, the terminal groups may be suitably modified by reacting the initially produced block polymer to introduce specific terminal groups (e.g., a cartilage-binding domain (e.g., a peptide-containing group)) that facilitate binding of the block copolymer to the desired biological tissue. In some embodiments, a cartilage-binding domain is attached to the block copolymer via an -S- linker, such as in the form of RS-, wherein R may be the cartilage-binding domain. In certain embodiments, the compound represented by formula (1) has terminal groups R and R', which may be as shown in formula (2):

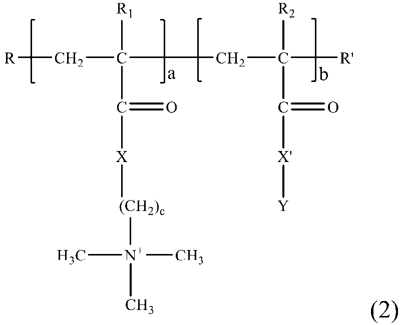

In certain embodiments, R may be as shown in formula (3):

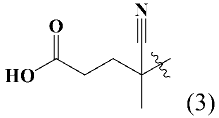

In certain embodiments, R' may be as shown in formula (4):

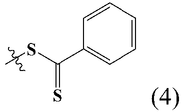

Moreover, although also not shown in formula (1), the total positive charge of quaternary ammonium groups in the copolymer represented by formula (1) is counterbalanced by an equivalent amount of total negative charge provided by anions associated with the quaternary ammonium groups. The anion may be selected from any acceptable species for use in organisms, such as a halide (e.g., chloride, bromide, or iodide), carbonate, bicarbonate, sulfate, bisulfate, bisulfite, carboxylate (e.g., acetate, propionate, butyrate, maleate, and citrate), and sulfonate (e.g., present between X and the quaternary ammonium group shown in formula (1)). In some embodiments, the anion is chloride, bromide, or iodide. In some embodiments, the anion is chloride.

The block copolymer represented by formula (1) described in the present application may include the compounds shown in the table below:

| No. | Structural formula |
|---|---|
| (5) | 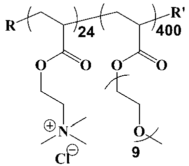 |
| (6) | 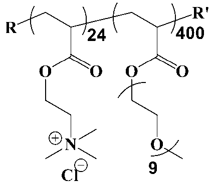 |
| (7) | 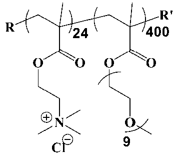 |
| (8) | 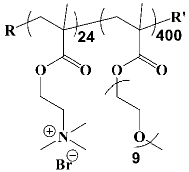 |
| (9) | 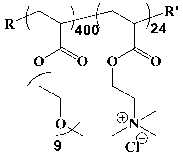 |
| (10) | 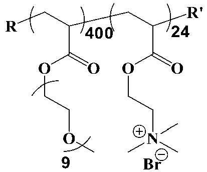 |
| (11) | 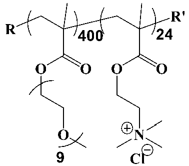 |
| (12) | 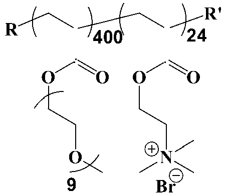 |
| (13) | 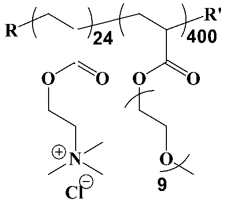 |
| (14) | 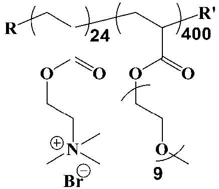 |
| (15) | 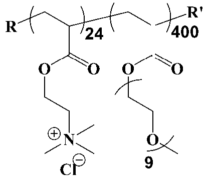 |
| (16) | 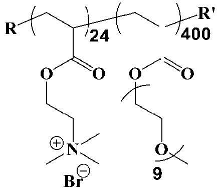 |
| (17) | 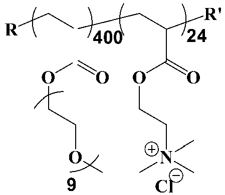 |
| (18) | 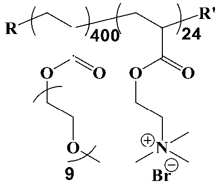 |
| (19) | 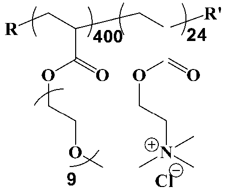 |
| (20) | 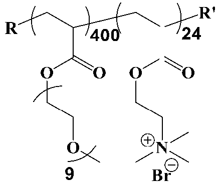 |

The block copolymer described in the present application may be synthesized by any suitable polymerization method. In a specific embodiment, the block copolymer is synthesized by reversible addition-fragmentation chain-transfer (RAFT) polymerization, which is well known in the art. In the RAFT process, a first polymer block is produced by reacting a first functionalized vinyl monomer with a RAFT transfer agent in the presence of a polymerization initiator. Then, in the presence of a polymerization initiator, the first polymer block is reacted with a second functionalized vinyl monomer, so as to attach a block of polymerized second functionalized vinyl monomer to the first polymer block.

In another aspect, the present application provides a composition, comprising one or more of the block copolymers described in the present application. In certain embodiments, the composition may exhibit a lubricating effect. In certain embodiments, the composition may enhance the lubricity or impart an appropriate level of lubricity to a biological tissue. In certain embodiments, the composition may play a lubricating role within a biological tissue or between biological tissues. The biological tissue is selected from a joint, a bone, an ocular tissue, a nasal tissue, a tendon, a tendon sheath, and a vaginal tissue.

In certain embodiments, the composition may be a pharmaceutical composition, and may further comprise one or more pharmaceutically acceptable carriers. The pharmaceutical composition may further comprise one or more adjuvants, such as stabilizers, surfactants, salts, buffers, additives, or combinations thereof, all of which are well known in the pharmaceutical field. The stabilizer may be, for example, an oligosaccharide (e.g., sucrose, trehalose, lactose, or dextran), a sugar alcohol (e.g., mannitol), or a combination thereof. The surfactant may be any suitable surfactant, including, for example, those containing polyalkylene oxide units (e.g., Tween 20, Tween 80, or Pluronic F-68), which are typically included in an amount ranging from about 0.001% (w/v) to about 10% (w/v). The salt or buffer may be any suitable salt or buffer, such as sodium chloride or sodium or potassium phosphate, respectively. Some examples of additives include, for example, glycerol, benzyl alcohol, and 1,1,1-trichloro-2-methyl-2-propanol (e.g., chloroacetone or chlorobutanol). If desired, the pH of the solution can be appropriately adjusted by including a pH adjuster. Pharmaceutical compositions and formulations for topical administration may include transdermal patches, ointments, lotions, creams, gels, drops, suppositories, sprays, liquids, and powders. Conventional pharmaceutical carriers, aqueous, powdered or oily bases, thickeners, etc., may be necessary or required. The pharmaceutical formulation may be in the form of a sterile aqueous solution containing one or more buffers, diluents, and/or other suitable additives, such as, but not limited to, penetration enhancers and carrier compounds.

When a biological tissue slides relative to an identical tissue or other substances, an increase in the level of lubricity generally corresponds to a decrease in the level of friction (i.e., the coefficient of friction, or COF). The coefficient of friction can be measured using a tribometer, evaluating surface lubrication through linear oscillation of the sample at variable speeds (typically 0.1, 0.3, 1, 3, and 10 mm/s) and variable compressive normal stresses (typically 250 to 300 kPa).

In another aspect, the present application provides use of the block copolymer described in the present application and/or the composition described in the present application in enhancing the lubricity of a biological tissue or imparting an appropriate level of lubricity to a biological tissue.

In another aspect, the present application provides a method for imparting an appropriate level of lubricity to a biological tissue, comprising bringing the biological tissue into contact with the block copolymer described in the present application and/or the composition described in the present application to impart the appropriate lubricity. In certain embodiments, the appropriate lubricity may comprise an increase in the level of lubricity.

In certain embodiments, the increase in the level of lubricity may comprise a decrease in the level of friction. For example, compared to before the contact of the block copolymer described in the present application and/or the composition described in the present application with the biological tissue, when the biological tissue slides relative to an identical tissue or other substances, the level of friction is decreased by at least about 5%, at least about 10%, at least about 20%, at least about 30%, at least about 40%, at least about 50%, at least about 60%, at least about 70%, at least about 80%, at least about 90%, or more. The level of friction can be measured by means commonly used in the art. For example, the level of friction can be reflected by the determination of the coefficient of friction.

In certain embodiments, the appropriate lubricity may comprise wear protection. In certain embodiments, the appropriate lubricity may comprise prevention of wear. For example, the wear protection or prevention of wear may comprise slowing down or reducing the loss of mass or volume resulting from wear between biological tissues. For example, compared to before the contact of the block copolymer described in the present application and/or the composition described in the present application with the biological tissue, when the biological tissue slides relative to an identical tissue or other substances, the loss of mass or volume produced by the biological tissue within the same period of time may be reduced by at least about 5%, at least about 10%, at least about 20%, at least about 30%, at least about 40%, at least about 50%, at least about 60%, at least about 70%, at least about 80%, at least about 90%, or more. The effect of wear protection or prevention of wear can be tested by means commonly used in the art, for example, by a friction and wear test.

The biological tissue is selected from a joint, a bone, an ocular tissue, a nasal tissue, a tendon, a tendon sheath, and a vaginal tissue.

In another aspect, the present application relates to a method for imparting lubricity to a biological tissue (such as a joint, cartilage, and bone) by using the biomimetic block copolymer described in the present application. In the case of bone, the biomimetic copolymer is capable of reducing the discomfort, pain, and additional damage caused by direct bone-to-bone contact that sometimes occurs in the advanced stages of osteoarthritis. According to the method, the biological tissue is brought into contact with a sufficient (i.e., effective or therapeutically effective) amount of any of the biomimetic block copolymers described in the present application to enhance the lubricity or impart an appropriate level of lubricity to the biological tissue.

A therapeutically effective amount may be administered to a patient in one or more doses, the dose(s) being sufficient to mitigate, alleviate, stabilize, reverse, or slow the progression of the disease or disorder, or to reduce the pathological consequences of the disease or disorder, or to reduce the symptoms of the disease or disorder. The alleviation or reduction need not be permanent, but can be sustained for a period of time, such as at least one hour, at least one day, or at least one week or more. The effective amount is generally determined by a physician on a case-by-case basis, which falls within the skills of those skilled in the art. In determining an appropriate dose to achieve an effective amount, several factors are generally considered. These factors include the age, sex and body weight of the patient, the disorder being treated, the severity of the disorder, and the route, dosage form and regimen of administration, as well as the desired outcome. In certain embodiments, the therapeutically effective amount is an amount capable of effectively treating osteoarthritis, achieving pain relief within a period of time, improving joint mobility and flexibility, reducing friction in the joint, or improving other acceptable indicators for osteoarthritis. The therapeutically effective amount can be adjusted by using concentration and injection volume. For example, the block copolymer described in the present application may be used at a concentration of 0.001 mg/mL to 100 mg/mL. For example, the block copolymer described in the present application may be used at a concentration of 0.1 mg/mL to 100 mg/mL. For example, the block copolymer described in the present application may be used at a concentration of 1-5 mg/mL. For example, the block copolymer described in the present application may be used at a concentration of 1 mg/mL to 100 mg/mL. For example, the block copolymer described in the present application may be administered at a dose of about 0.1 mL to about 10 mL. For example, the block copolymer described in the present application may be administered at a dose of about 0.1 mL to about 3 mL. In an exemplary embodiment, the dose level ranges from a volume of 0.1 mL to 10 mL injected at a concentration of 0.1-100 mg/mL (for humans), and more typically from a volume of 0.1 mL to 3 mL injected at a concentration of about 1-5 mg/mL. In certain embodiments, the therapeutically effective amount is an amount capable of effectively reducing the level of friction in the biological tissue. In certain embodiments, the therapeutically effective amount is an amount capable of effectively providing wear protection in biological tissues. In certain embodiments, the therapeutically effective amount is an amount capable of effectively providing lubricity in the biological tissue to be lubricated.

The biological tissue to be lubricated may be brought into contact with any of the block copolymers described in the present application by any method well known in the medical field. The block copolymer can be delivered directly into or onto the biological tissue, or indirectly into the biological tissue surrounding the tissue to be lubricated, via, for example, injection, infusion, implantation, spraying, or coating, thereby bringing the biological tissue into contact with the block copolymer. In general, contact with the biological tissue refers to delivering the block copolymer to the tissue by any method that uses the copolymer to coat the surface of the tissue or soak the tissue. In certain embodiments, the composition is injected or infused into the joint space to come into contact with the tissue, thereby resulting in coating of the cartilage and/or meniscus present in the joint space. Moreover, the volume used depends at least in part on the type of tissue being contacted, and on whether a space is to be filled or a surface is to be coated, as can be determined by those skilled in the medical field.

In certain embodiments, the block copolymer is injected or infused into or onto an arthritic or injured joint or bone to improve the lubricity of the joint or bone. In this way, the copolymer provides boundary lubrication. The treatment can be particularly used for treating or preventing osteoarthritis. The treatment of osteoarthritis or injured joints, cartilage, or bones preferably results in the following outcomes: alleviation of symptoms, improvement in mobility, reduction in joint pain, and overall inhibition of disease progression, or prevention in the case of joint injury. The method may further comprise administering one or more of the block copolymers described above, and simultaneously or sequentially administering another composition that functions to enhance or work in conjunction with the block copolymer and falls outside the scope of the block copolymers described in the present application. The enhancing (i.e., auxiliary) composition may be selected from, for example, hyaluronic acid, lubricin, synovial fluid, glycosaminoglycans, and other adjuvants. These additional agents may also be administered by, for example, injection or infusion. In certain embodiments, these additional agents can act synergistically with one or more of the block copolymers described above to provide enhanced lubrication and wear protection.

In a specific embodiment, the biological tissue is a joint, cartilage, or bone, more typically an injured or arthritic joint, cartilage, or bone. In certain embodiments, the joint is a weight-bearing joint, such as a hip joint, a knee joint, or an ankle joint. Many different joints can benefit from an increase in the level of lubricity, including the shoulder, elbow, wrist, hand, finger, and toe joints. However, the lubricated biological tissue is not limited to joints, cartilage, and bones. Other biological tissues, including ocular tissue, nasal tissue, and vaginal tissue, can be lubricated by using the disclosed block copolymer. Therefore, by using the block copolymer described in the present application, a variety of disorders other than those associated with joints, cartilage, and bones can be treated. Some of these other disorders include, for example, dry eye disease, dry nose, postmenopausal vaginal dryness, carpal tunnel syndrome, etc. Those skilled in the medical field can determine the appropriate delivery routes and methods for contact with particular biological tissues. For example, for dry eyes, contact can be achieved by drop instillation; for dry nose, contact can be achieved by nasal spray; for carpal tunnel syndrome, contact can be achieved by injection near or around the inflamed tendon and sheath; and for postmenopausal vaginal dryness, a pill, lozenge, or suppository can be placed or implanted in the vagina. Therefore, the method can be used to achieve boundary-mode lubrication in any of various biological tissues that can benefit from additional lubrication.

Without being bound by any theory, the following examples are intended only to illustrate the fusion proteins, preparation methods, uses, etc., of the present application, and are not intended to limit the scope of the invention of the present application.

### Examples

### Example 1. Synthesis of Block Copolymers

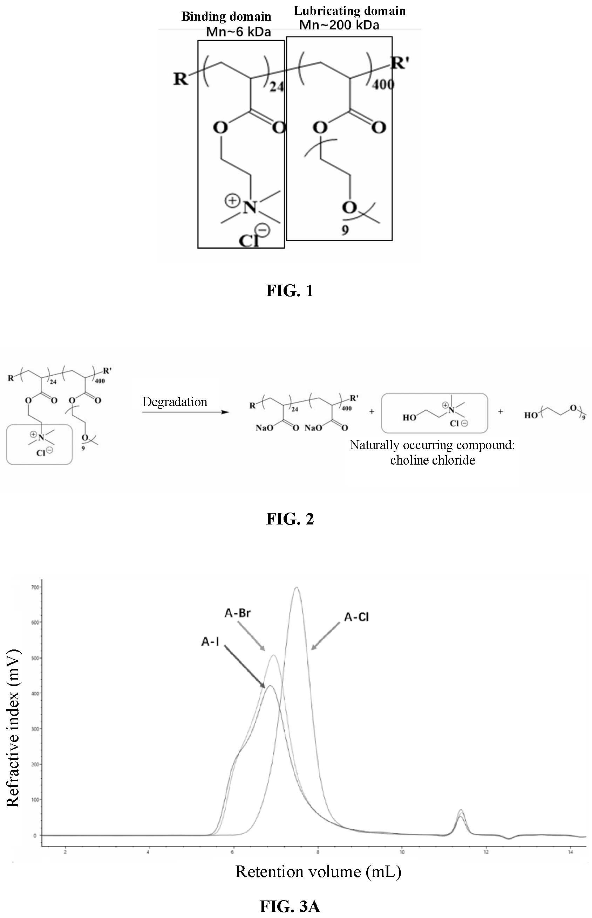

To mimic the structure of lubricin, a diblock copolymer was prepared in the present application, the copolymer comprising a larger lubricating block (Mn ~ 200 kDa) that mimics the mucin-like domain of lubricin and a smaller cartilage-binding block (Mn ~ 3-10 kDa) that mimics the C-terminal domain. An exemplary composition showing the two mimetic domains is provided in FIG. 1. The lubricating domain of the diblock copolymer shown in FIG. 1 comprises a poly(acrylic acid) main chain grafted with polyethylene glycol (PEG) brushes. The aforementioned characteristics contribute to the hydration and resistance to compression of the polymer. The binding domain of the diblock copolymer shown in FIG. 1 comprises a poly(acrylic acid) backbone with quaternary ammonium side groups that have non-specific interactions with negatively charged cartilage components (such as aggrecan).

The general synthetic route of the block copolymer described in the present application is as follows: 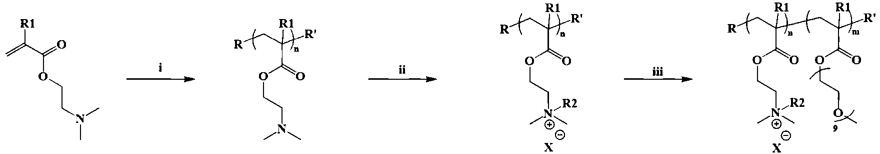 wherein R1 is hydrogen or methyl, R2 is methyl or ethyl, and X is Cl, Br, or I; n is 24, and m is 400; R is as shown in formula (3): 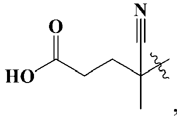 and R' is as shown in formula (4):

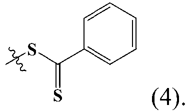

In some embodiments, the block copolymer described in the present application can be synthesized via a shorter synthetic route, which requires only two steps when the counterion is chloride, as described in the synthetic route below:

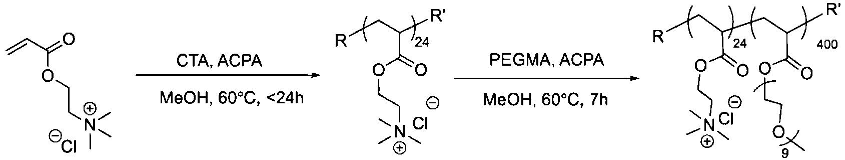

Specifically, a total of 6 compounds were synthesized in Example 1, and their structures are shown in the following figure. The differences between them were mainly reflected in the presence or absence of -CH₃ substitution on the main chain, and the counterions of the quaternary ammonium salts being different halide ions, i.e., chloride, bromide, and iodide ions.

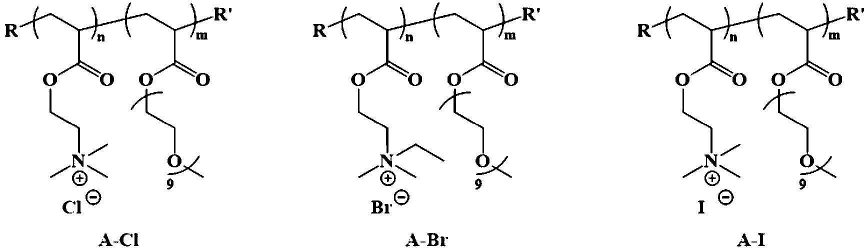

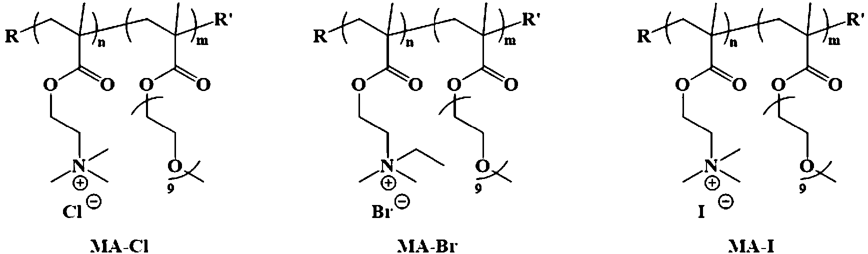

### 1.1. Synthesis of intermediates

In order to form the block copolymer, it was first necessary to synthesize key intermediates, the corresponding structures of which are shown below:

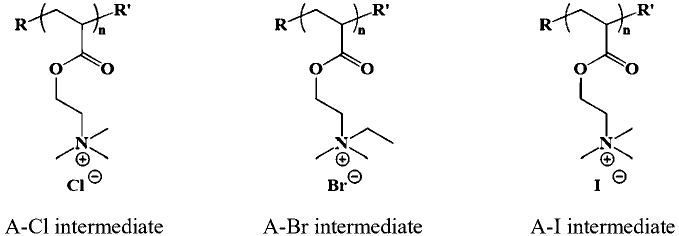

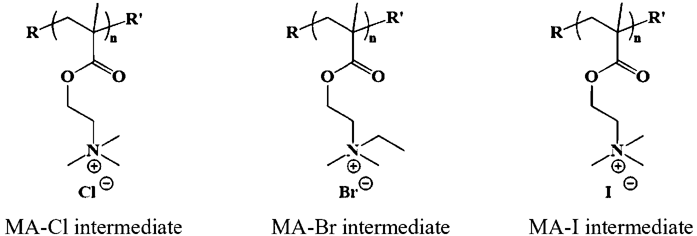

The above key intermediates could be synthesized in a total of two steps according to the general synthetic route. The A-I intermediate was taken as an example:

### Reaction i: Synthesis of poly(2-(dimethylamino)ethyl acrylate) (1)

2-(Dimethylamino)ethyl acrylate (DMAEA) (4.30 g, 30 mmol) was added to a 5 mL anisole solution containing 14.0 mg (0.05 mmol) of 4,4'-azobis(4-cyanopentanoic acid) (ACPA) and 139.5 mg (0.5 mmol) of 4-cyanopentanoic acid dithiobenzoate (CPADB). The mixture was degassed with nitrogen for 30 minutes and then heated to 70 °C for 48 hours. Then, the reaction was quenched by freezing at - 80 °C, and the residue was purified by precipitation induced by the addition of n-hexane (repeated 5 times). The structure of the purified product was confirmed by 1H NMR.

These reaction conditions were suitable for the generation of the A-Cl and A-Br intermediate precursors. During the synthesis of the MA-Cl/Br/I intermediate precursors, the reaction substrate needed to be replaced with the corresponding monomer, 2-(dimethylamino)ethyl methacrylate (DMAEMA).

### Reaction ii: Synthesis of quaternary ammonium salt derivative of poly(2-(dimethylamino)ethyl acrylate) (2)

At 0 °C, 4 mL of iodomethane was added dropwise to a 15 mL acetone solution containing 1.6 g of (1). The mixture was stirred at room temperature for 48 hours and then quenched by evaporating the solvent using a stream of nitrogen. The residue was dissolved in methanol, and the product was purified by precipitation induced by the addition of acetone (repeated 3 times). The structure of the purified product (2) was confirmed by 1H NMR and GPC.

These reaction conditions were suitable for the generation of the A-I and MA-I intermediates. During the synthesis of other precursors, the reaction substrate needed to be replaced with the corresponding haloalkane.

### 1.2. Synthesis of final product

After obtaining intermediate (2), methoxy-terminated PEG (9)-acrylate (7.2 mmol) or methoxy-terminated PEG (9)-methacrylate (7.2 mmol) was added to a methanol solution containing 0.009 mmol of key intermediate (2) and 0.5 mg (0.0018 mmol) of an initiator. The mixture was degassed with nitrogen, and then heated to 50-65 °C and allowed to react for 1-8 hours. Then, the reaction was quenched by freezing at -80 °C, and the residue was purified by precipitation induced by the addition of n-hexane (repeated 5 times). The structure of the purified product was confirmed by 1H NMR and GPC.

### Example 2. Characterization of Block Copolymers

### 2.1. Characterization of block copolymer properties

The products obtained in Example 1 were characterized by GPC. The test conditions for gel permeation chromatography were as follows: mobile phase: 0.1 M triethylamine and 1% glacial acetic acid solution (HPLC grade); flow rate: 0.5 mL/min; temperature: 30 °C; gel chromatography column: TSKgel G5000PWXL-CP, 7.8 mm ID × 30 cm. This method determined the absolute molecular weight of the samples by refractive index and small-angle light scattering measurements.

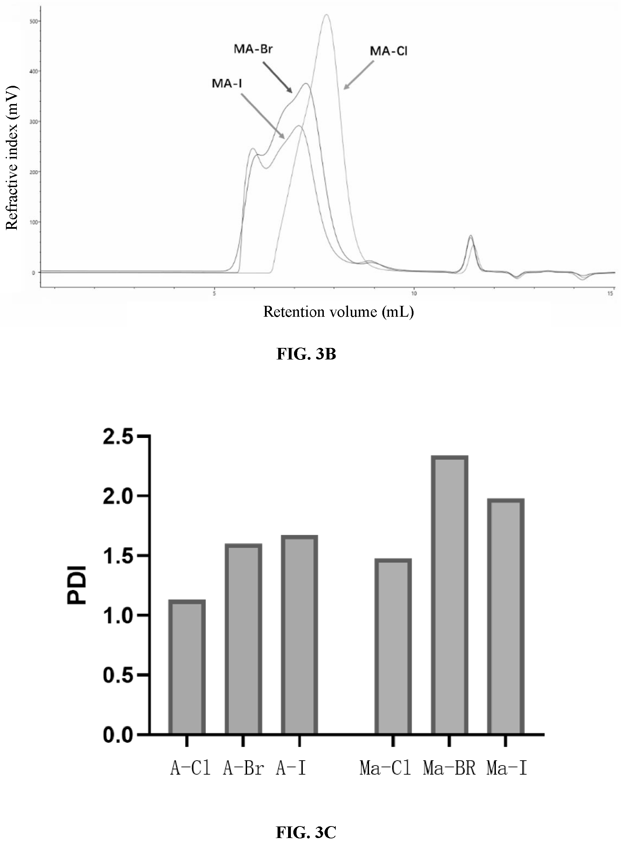

After purification of the final products, it was found by GPC characterization that among compounds of the same type, the PDI value was influenced by the type of the counterion, following the trend: Cl < Br ≈ I. This trend was observed in both compounds without methyl substitution on the main chain and compounds with methyl substitution on the main chain. The results are shown in FIGs. 3A-C. A summary of the data of FIGs. 3A-3C is shown in Table 1:

**Table 1**

| | A-Cl | A-Br | A-I | MA-Cl | MA-Br | MA-I |
|---|---|---|---|---|---|---|
| Mn | 249 kDa | 512 kDa | 676 kDa | 430 kDa | 535 kDa | 1013 kDa |
| Mw | 282 kDa | 820 kDa | 1133 kDa | 636 kDa | 1253 kDa | 2007 kDa |
| PDI | 1.133 | 1.600 | 1.677 | 1.479 | 2.34 | 1.981 |

This example illustrates that the block copolymers described in the present application have a more uniform molecular weight distribution.

### 2.2. Evaluation of lubricating ability of block copolymer on different biological tissues

This example illustrates that the block copolymer described in the present application exhibits successful lubrication effects on different material interfaces.

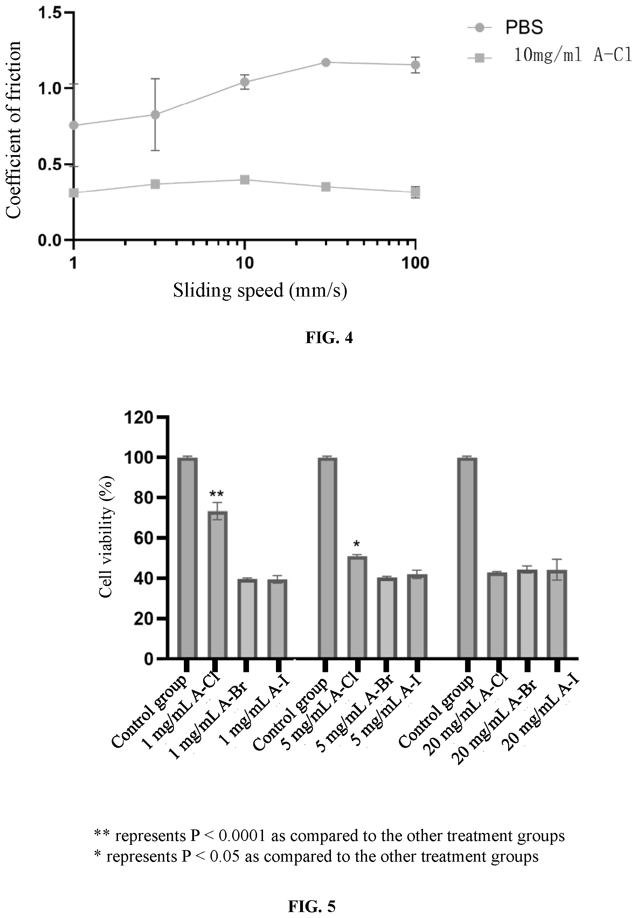

To evaluate the diblock copolymer as a synthetic lubricant, an Anton Paar M302e rheometer and its T-PID/44 tribology module were used to assess the tribological behavior of the copolymer. The tribology module employed a PDMS cylinder with a diameter of 6 mm and a smooth glass surface as interface materials. Under the boundary conditions of 25 °C and a 5 N normal force, the coefficient of friction (COF) was measured at different sliding speeds (1-100 mm/s) in a PBS solution and a PBS solution containing the block copolymer (10 mg/mL). The *in vitro* lubrication test results indicate that the block copolymer exhibited good lubrication effects at different speeds, reducing the COF by 60% or more, from a range of 0.756-1.155 down to 0.316-0.398 (FIG. 4).

### Example 3. Cytotoxicity Assay of Intermediates

This example illustrates that the block copolymers described in the present application have ameliorated cytotoxicity and improved biocompatibility.

In this example, the *in vitro* toxicity of intermediates containing different counterions to human articular chondrocytes (HC-a) was investigated. Considering that these intermediates will ultimately become one of the important structures of the final products, these results can, to some extent, reflect the toxicity trend of the final products with different structures.

The assay procedures were as follows:
HC-a cells in the logarithmic growth phase were seeded into 96-well plates (4000 cells/well) and cultured at 37 °C with 5% CO₂ for 12 hours. After treatment with different concentrations of intermediates (1 mg/mL, 5 mg/mL, and 20 mg/mL) for 24 hours, the cell viability was measured separately: After the cell supernatant was removed, 100 µL of a fresh DMEM medium containing 10% CCK8 was added to each well, followed by incubation at 37 °C with 5% CO₂ for 2 hours. Then, the absorbance of each well was measured at a wavelength of 450 nm. The results obtained are shown in FIG. 5.

As shown in the results in FIG. 5, the A-Cl intermediate showed better *in vitro* cell safety compared to the intermediates containing other halide ions. At the 1 mg/mL concentration, the cell viability in the A-Cl intermediate sample-treated group far exceeded that in the A-Br/I intermediate sample-treated groups, and was closer to the result in the negative control group. When the concentration was increased to 5 mg/mL, although the cell activity in the A-Cl intermediate sample group showed a certain decrease, it still statistically exceeded that in the other sample groups. The advantage in cell viability of the A-Cl intermediate over the other samples persisted until the sample concentration was increased to 20 mg/mL.

### Example 4. Stability Test

In this example, the stability of different compounds under moist heat sterilization conditions was investigated.

The test procedures were as follows: First, 6 types of compounds with different counterions and different backbone structures were each dissolved in 1× PBS at a concentration of 30 mg/mL. After complete dissolution, the samples were separately transferred into vials for moist heat sterilization treatment. The sterilization was carried out at a temperature of 121 °C for a duration of 15 minutes. After the sterilization was completed, the sample solutions were lyophilized and then sent for NMR detection, and the proportion of hydrolysis products generated during the moist heat sterilization was calculated by integrating the corresponding characteristic peaks.

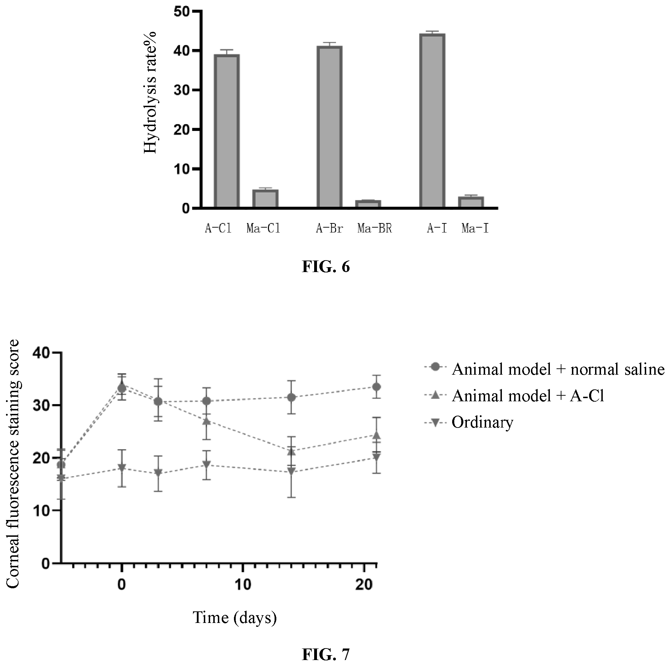

As shown in the results in FIG. 6, when the backbone structure had methyl substitution on the main chain, the hydrolysis products were greatly reduced compared to the structure without methyl substitution on the main chain. This conclusion was correspondingly reflected in all three types of counterions: Cl/Br/I. Therefore, the inclusion of methyl in the backbone structure could improve the stability of the block compound.

### Example 5. Animal Model of Dry Eye Disease

Based on the good cytotoxicity results of A-Cl, its potential therapeutic effect in a mouse model of dry eye disease was further verified in this example.

The experimental procedures were as follows:
In this experiment, C57BL/6J mice were used. Prior to the start of the experiment, the animals had been acclimated to the animal facility environment for at least 5 days. After the start of the experiment (D1-D26), the molded mice were placed in a low-humidity environment (≤ 40% RH) while receiving twice-daily subcutaneous injections of scopolamine hydrobromide (0.75 mg/mL) in the dorsal neck region, with 0.3 mL injected each time and an interval of 6 hours between injections. After day 5 of modeling (D6-D26), eye drop administration was performed twice daily for 21 consecutive days. The corneal fluorescence staining scores are shown in FIG. 7. It can be seen that from day 14 onwards, A-Cl statistically significantly reduced the corneal fluorescence staining score, showing no statistically significant difference compared to the healthy control group, and this effect persisted at least until day 21. Throughout the entire administration period, no significant side effects or irritation were observed, and the body weight of the mice was not affected.

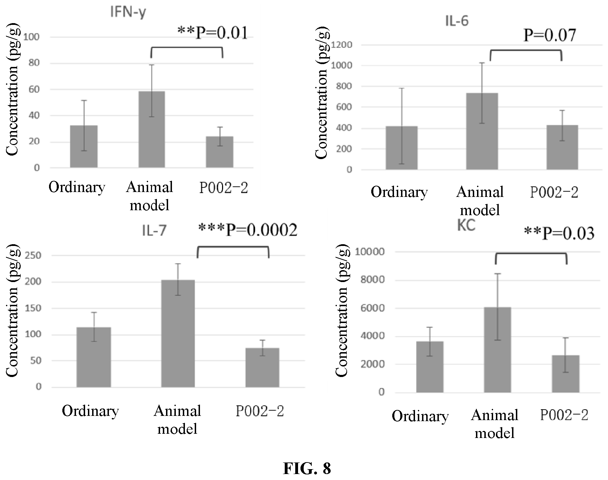

After the end of the experiment, the eyeballs of the corresponding animals were enucleated, and the cornea and conjunctiva of each eye were collected and pooled for storage at -80 °C. Subsequently, the protein content in the cornea and conjunctiva was determined using the BCA method, and the levels of multiple immune factors were measured using a multiplex protein assay kit (t-multiplex-m18h) suitable for immunoassay, as shown in FIG. 8. The results show that at least 4 immune factors were significantly reduced in the test substance groups compared to the model control group, further validating the therapeutic effect of P002 in the dry eye disease model.

While what are presently considered to be preferred embodiments of the present disclosure have been shown and described, various changes and modifications can be made by those skilled in the art, and these changes and modifications are still within the scope of the present disclosure as defined by the appended claims.

## Claims

1. A block copolymer having the following structure: 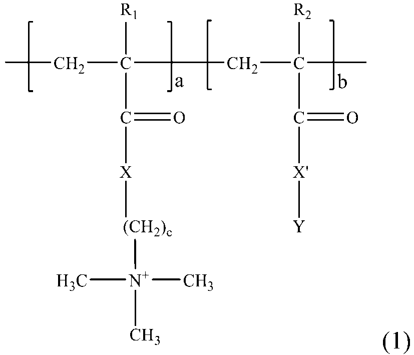 wherein:
R1 and R2 are each independently selected from hydrogen and methyl;
X and X' are each independently selected from -NR'-, -O-, and a bond, wherein R' is selected from a hydrogen atom and a group having at least 1 and at most 6 carbon atoms;
Y is selected from a polyalkylene glycol, a saccharide, and a polyol;
subscript a is an integer of at least 3, subscript b is at least twice a, and
subscript c is an integer of at least 1;
according to the laws of chemistry, the block copolymer is terminated at each terminus with a terminal group, and the total positive charge of quaternary ammonium groups in the copolymer is counterbalanced by an equal magnitude of total negative charge provided by anions associated with the quaternary ammonium groups.

2. The block copolymer according to claim 1, wherein subscript b is an integer greater than 40.

3. The block copolymer according to any one of claims 1-2, wherein subscript b is an integer of at least 50.

4. The block copolymer according to any one of claims 1-3, wherein subscript b is an integer of at least 100.

5. The block copolymer according to any one of claims 1-4, wherein subscript b is an integer of 200-400, and subscript a is an integer of 20-96.

6. The block copolymer according to any one of claims 1-5, wherein subscript b is 400, and subscript a is 24.

7. The block copolymer according to any one of claims 1-6, wherein Y comprises a polyalkylene glycol.

8. The block copolymer according to claim 7, wherein the polyalkylene glycol comprises polyethylene glycol.

9. The block copolymer according to any one of claims 1-8, wherein the saccharide comprises a monosaccharide.

10. The block copolymer according to any one of claims 1-9, wherein the saccharide comprises at least two monosaccharide units.

11. The block copolymer according to any one of claims 1-10, wherein the polyalkylene glycol, the saccharide, or the polyol is composed of at least 2 and at most 500 monomer units.

12. The block copolymer according to any one of claims 1-11, wherein the polyalkylene glycol, the saccharide, or the polyol is composed of at least 2 and at most 200 monomer units.

13. The block copolymer according to any one of claims 1-12, wherein the polyalkylene glycol, the saccharide, or the polyol is composed of at least 2 and at most 100 monomer units.

14. The block copolymer according to any one of claims 1-13, wherein the polyalkylene glycol, the saccharide, or the polyol is composed of at least 2 and at most 20 monomer units.

15. The block copolymer according to any one of claims 1-14, wherein at least one of the terminal groups is sulfhydryl.

16. A composition, comprising the block copolymer according to any one of claims 1-15.

17. The composition according to claim 16, comprising one or more pharmaceutically acceptable carriers.

18. Use of the block copolymer according to any one of claims 1-15 and/or the composition according to any one of claims 16-17 in enhancing the lubricity of a biological tissue or imparting an appropriate level of lubricity to a biological tissue.

19. Use of the block copolymer according to any one of claims 1-15 and/or the composition according to any one of claims 16-17 in treating dry eye disease.

20. Use of the block copolymer according to any one of claims 1-15 and/or the composition according to any one of claims 16-17 in preparing a medicament for treating dry eye disease.

21. A method for imparting an appropriate level of lubricity to a biological tissue, comprising bringing the biological tissue into contact with the block copolymer according to any one of claims 1-15 and/or the composition according to claim 16 to impart the appropriate lubricity.

22. The use according to claim 17 or the method according to claim 18, wherein the biological tissue is selected from a joint, a bone, an ocular tissue, a nasal tissue, a tendon, a tendon sheath, and a vaginal tissue.
